# EUROPEAN PATENT APPLICATION

(11) **EP 3 581 096 A1**
(43) Date of publication of application: **18.12.2019**
(21) Application number: 19177957.8
(22) Date of filing: 03.06.2019
(51) Int. Cl.: A61B 5/00, A61B 5/1455

(54) **OPTO-MECHANICAL DESIGN OF BIOSENSOR FOR HUMAN BODY SIGNAL DETECTION**

(30) Priority: 11.06.2018 US 201862683090 P; 27.05.2019 US 201916423133
(71) Applicant: MediaTek Inc., Hsin-Chu 300 (TW)
(72) Inventor: CHEN, Yu-Wen, Hsin-Chu 30078 (TW); KUO, Jing-Lin, Hsin-Chu 30078 (TW); ZOU, Teng-Feng, Hsin-Chu 30078 (TW)
(74) Representative: Haseltine Lake Kempner LLP

(57) **Abstract**

The present invention provides an electronic device comprising at least two light emitters and at least one photo detector. The at least two light emitters comprise a first light emitter and a second light emitter, and the first light emitter emits light whose wavelength is greater than 1000 nanometer (nm) ; the at least one photo detector is configured to receive the light reflected by a human body to generate a plurality of physiological signals, wherein the physiological signals are arranged to obtain at least two physiological features of the human body.

## Description

This application claims the priority of US Provisional Application No. 62/683,090, filed on June 11th, 2018.

### Background

Recently, personal biosensors become popular for providing physiological information at all time for the reference to the user. However, the current designs of the biosensors may not provide sufficient information to the user.

### Summary

It is therefore an objective of the present invention to provide Opto-mechanical design of the biosensor, which can effectively and accurately measure one or more specific layers of a human body, to solve the above-mentioned problems.

According to one embodiment of the present invention, a biosensor comprising at least two light emitters and at least one photo detector is disclosed. The at least two light emitters comprise a first light emitter and a second light emitter, and the first light emitter emits light whose wavelength is greater than 1000 nanometer (nm); the at least one photo detector is configured to receive the light reflected by a human body to generate a plurality of physiological signals, wherein the physiological signals are arranged to obtain at least one physiological feature of the human body.

According to another embodiment of the present invention, an electronic device comprising a biosensor and a processing circuit is disclosed, wherein the biosensor comprises at least two light emitters and at least one photo detector. The at least two light emitters comprise a first light emitter and a second light emitter, and the first light emitter emits light whose wavelength is greater than 1000 nm. The at least one photo detector is configured to receive the light reflected by a human body to generate a plurality of physiological signals. The processing circuit is configured to analyze the plurality of physiological signals to obtain at least one physiological feature of the human body.

These and other objectives of the present invention will no doubt become obvious to those of ordinary skill in the art after reading the following detailed description of the preferred embodiment that is illustrated in the various figures and drawings.

### Brief Description of the Drawings

FIG. 1 is a diagram illustrating an electronic device according to one embodiment of the present invention.
FIG. 2 shows three layers of the human body.
FIG. 3 is a diagram illustrating the biosensor according to a first embodiment of the present invention.
FIG. 4 is a diagram illustrating the biosensor according to a second embodiment of the present invention.
FIG. 5 is a diagram illustrating the biosensor according to a third embodiment of the present invention.

### Detailed Description

Certain terms are used throughout the following description and claims to refer to particular system components. As one skilled in the art will appreciate, manufacturers may refer to a component by different names. This document does not intend to distinguish between components that differ in name but not function. In the following discussion and in the claims, the terms "including" and "comprising" are used in an open-ended fashion, and thus should be interpreted to mean "including, but not limited to ...". The terms "couple" and "couples" are intended to mean either an indirect or a direct electrical connection. Thus, if a first device couples to a second device, that connection may be through a direct electrical connection, or through an indirect electrical connection via other devices and connections.

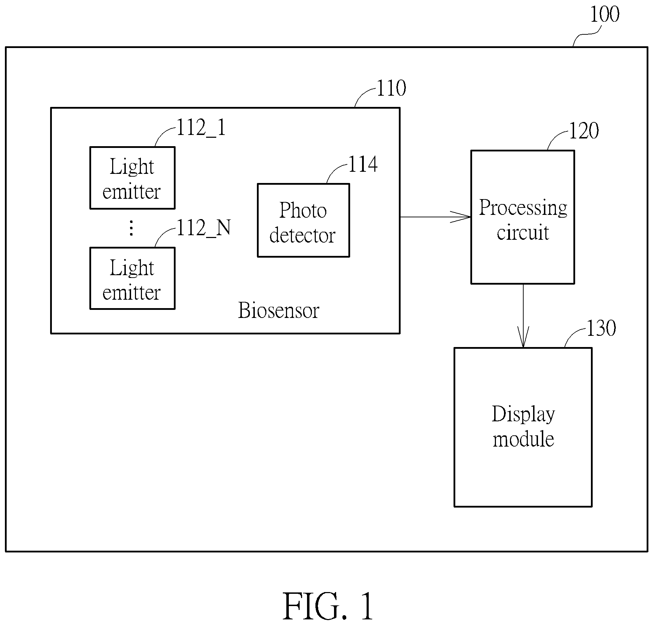

FIG. 1 is a diagram illustrating an electronic device 100 according to one embodiment of the present invention. As shown in FIG. 1, the electronic device 100 comprises a biosensor 110, a processing circuit 120 and a display module 130, wherein the biosensor 110 comprises a plurality of light emitters 112_1 - 112_N and at least one photo detector 114. In this embodiment, the electronic device 100 may be a smart phone, a pad, a tablet, a watch, an accessary or a wearable device.

In the operations of the electronic device 100, the biosensor 110 is used to contact to a human body such as a finger, and the light emitters 112_1 - 112_N emit the light to the human body, and the photo detector 114 receives the reflected light from the human body to generate a plurality of physiological signals to the processing circuit 120. Then, the processing circuit 120 analyzes the physiological signals to obtain one or more physiological features, and the physiological feature(s) is/are shown on a screen of the display module 130 for the reference to the user.

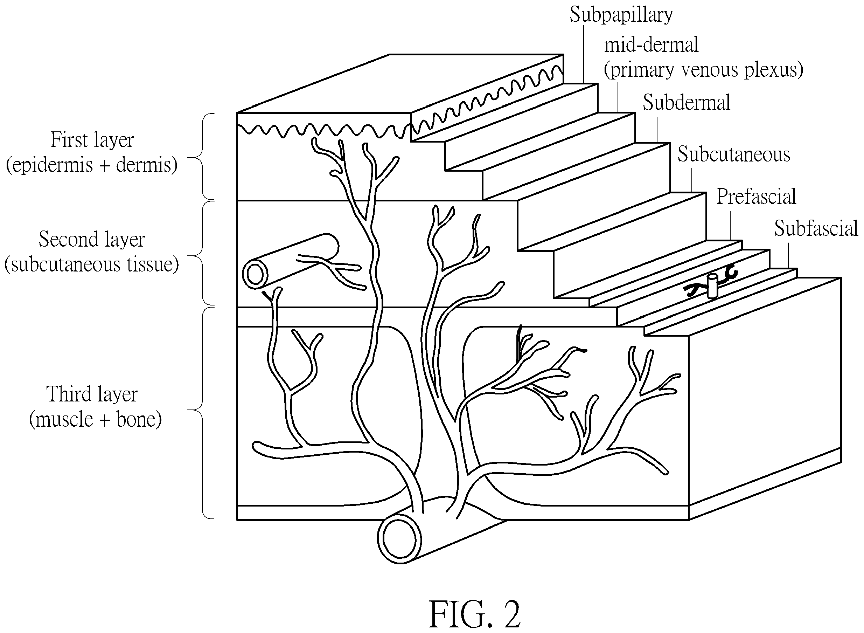

Regarding the biosensor 110, the light emitter 112_1 - 112_N have different wavelengths, and distances between the photo detector 114 and each of the light emitter 112_1 - 112_N are designed to make the photo detector 114 be able to receive the light reflected from a particular layer of the human body. Taking FIG. 2 as an example, a first layer of the human body is the epidermis and dermis layer, and the first layer comprises skin, subpapillary and mid-dermal (primarily venous plexus); a second layer of the human body is the subcutaneous tissue, and the second layer comprises subdermal, subcutaneous and prefascial; a third layer of the human body is the muscle/bone layer, and the third layer comprises the subfascial, muscle, internal artery and bone. The biosensor 110 is designed to make the photo detector 114 able to receive the light reflected from the needed layer, to effectively and accurately determine the physiological features of the human body.

Regarding the first layer shown in FIG. 2, the light emitter such as 112_1 may emit the light whose wavelength is ranging from 350 - 600 nm, and the distance between the light emitter 112_1 and the photo detector 114 is ranging from 0.1 - 10 millimeter (mm), so that the photo detector 114 can receive the light reflected from the first layer. The light emitter 112_1 may emit the light whose wavelength is ranging from 600 - 1100 nm, and the distance between the light emitter 112_1 and the photo detector 114 is ranging from 0.1 - 5 mm, so that the photo detector 114 can receive the light reflected from the first layer. In addition, the light emitter 112_1 may emit the light whose wavelength is ranging from 1100 - 2000 nm, and the distance between the light emitter 112_1 and the photo detector 114 is ranging from 0.1 - 10 mm, so that the photo detector 114 can receive the light reflected from the first layer.

Regarding the second layer shown in FIG. 2, the light emitter such as 112_1 may emit the light whose wavelength is ranging from 350 - 600 nm, and the distance between the light emitter 112_1 and the photo detector 114 is ranging from 0.1 - 10 mm, so that the photo detector 114 can receive the light reflected from the second layer. The light emitter 112_1 may emit the light whose wavelength is ranging from 600 - 1100 nm, and the distance between the light emitter 112_1 and the photo detector 114 is ranging from 5 - 15 mm, so that the photo detector 114 can receive the light reflected from the second layer. In addition, the light emitter 112_1 may emit the light whose wavelength is ranging from 1100 - 2000 nm, and the distance between the light emitter 112_1 and the photo detector 114 is ranging from 5 - 20 mm, so that the photo detector 114 can receive the light reflected from the second layer.

Regarding the third layer shown in FIG. 2, the light emitter such as 112_1 may emit the light whose wavelength is ranging from 350 - 600 nm, and the distance between the light emitter 112_1 and the photo detector 114 is ranging from 0.1 - 10 mm, so that the photo detector 114 can receive the light reflected from the third layer. The light emitter 112_1 may emit the light whose wavelength is ranging from 600 - 1100 nm, and the distance between the light emitter 112_1 and the photo detector 114 is ranging from 10 - 35 mm, so that the photo detector 114 can receive the light reflected from the third layer. In addition, the light emitter 112_1 may emit the light whose wavelength is ranging from 1100 - 2000 nm, and the distance between the light emitter 112_1 and the photo detector 114 is ranging from 15 - 50 mm, so that the photo detector 114 can receive the light reflected from the third layer.

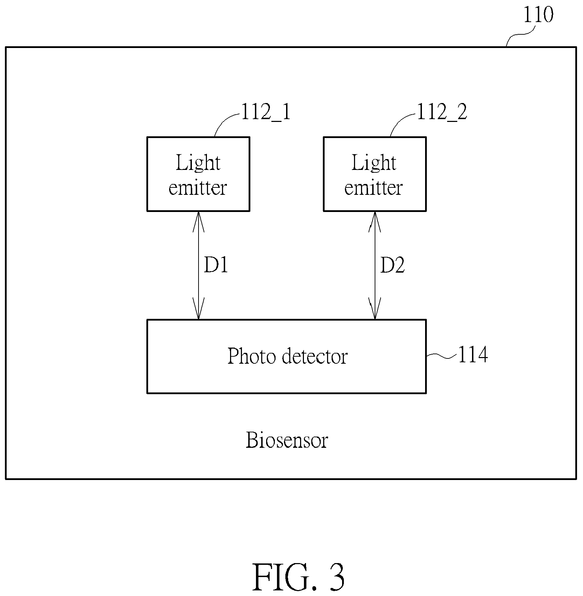

In one embodiment, the biosensor 110 comprises two light emitters 112_1 and 112_2 as shown in FIG. 3, the light emitters 112_1 and 112_2 are light-emitted diodes with 1500 nm and 1750 nm, respectively, and the distances between the photo detector 114 and the light emitters 112_1 and 112_2 are about 20 nm (i.e. D1∼20nm and D2∼20nm). By using the opto-mechanical design shown in FIG. 3, because the third layer is measured by using two different light emitters with different wavelengths, the photo detector 114 can receive the light reflected from the third layer to generate two physiological signals, and the processing circuit 120 may use the two physiological signals to accurately obtain the physiological features comprising neuron, protein, glucose, cholesterol, bilirubin, uric acid, lipid, water, lactate content in muscle, muscle content, muscle density, bone content or bone density.

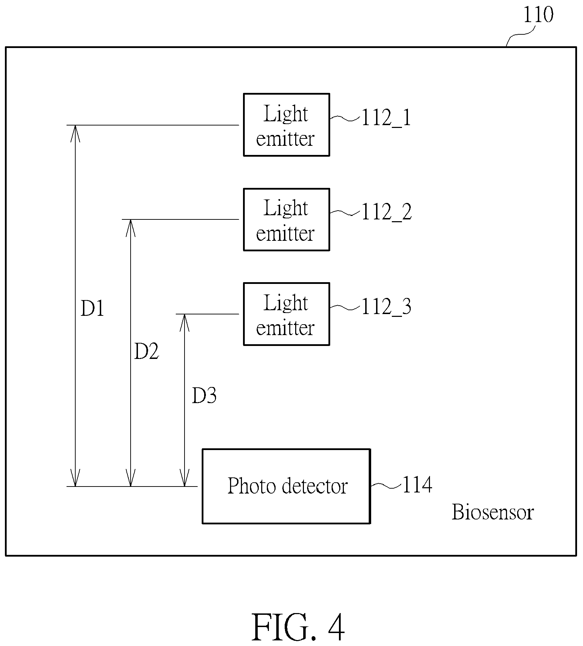

In another embodiment, the biosensor 110 comprises three light emitters 112_1 - 112_3 as shown in FIG. 4, the light emitters 112_1 - 112_3 are light-emitted diodes with 1550 nm, 1050 nm and 950 nm, respectively, the distance between the photo detector 114 and the light emitter 112_1 is about 20 mm (i.e. D1∼20 nm), the distance between the photo detector 114 and the light emitter 112_2 is about 15 mm (i.e. D2∼15 nm), and the distance between the photo detector 114 and the light emitter 112_3 is about 9 mm (i.e. D3∼9 nm). By using the opto-mechanical design shown in FIG. 4, because the second layer is measured by using different light emitters with different wavelengths, the photo detector 114 can receive the light reflected from the second layer to generate a plurality of physiological signals, and the processing circuit 120 may use the physiological signals corresponding to the second layer to accurately obtain the physiological feature (s) such as body water, fat content, fat density, neutron, protein or blood related content (e.g. glucose, cholesterol, bilirubin, uric acid or alcohol).

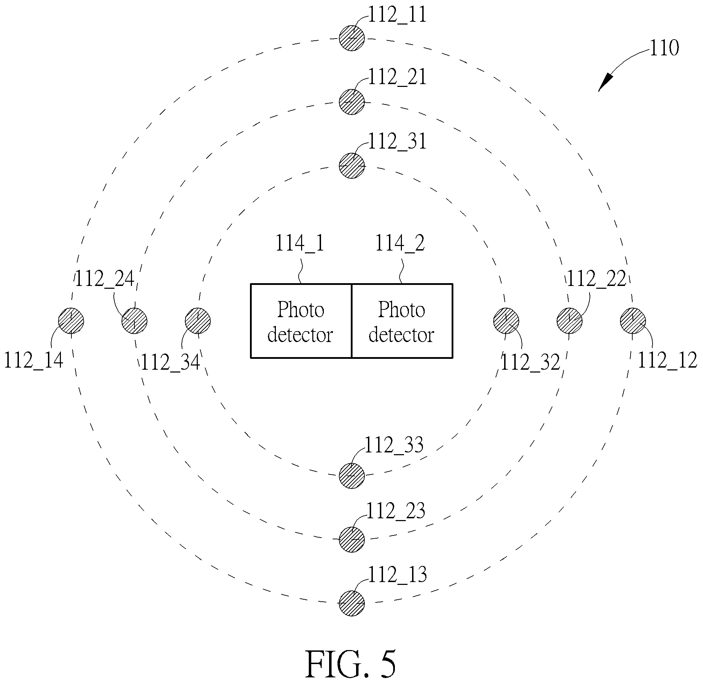

In another embodiment, the biosensor 110 comprises a plurality of light emitters 112_11 - 112_14, 112_21 - 112_24 and 112_31 - 112_34 and two photo detectors 114_1 and 114_2 as shown in FIG. 5, the light emitters 112_11 - 112_14 are light-emitted diodes with 1550 nm, and the light emitters 112_11 - 112_14 are arranged as a circle, and the distance between the photo detector 114_1/114_2 and the light emitters 112_11 - 112_14 is about 6 mm; the light emitters 112_21 - 112_24 are light-emitted diodes with 970 nm, and the light emitters 112_21 - 112_24 are arranged as a circle, and the distance between the photo detector 114_1/114_2 and the light emitters 112_21 - 112_24 is about 4 mm; and the light emitters 112_31 - 112_34 are light-emitted diodes with 860 nm, and the light emitters 112_31 - 112_34 are arranged as a circle, and the distance between the photo detector 114_1/114_2 and the light emitters 112_31 - 112_34 is about 2 mm. By using the opto-mechanical design shown in FIG. 5, because the first layer is measured by using different light emitters with different wavelengths, the photo detectors 114_1 and 114_2 can receive the light reflected from the first layer to generate a plurality of physiological signals, and the processing circuit 120 may use the physiological signals corresponding to the first layer to accurately obtain the physiological feature (s) such as skin quality (e.g. water, collagen, melanin, elastic fiber), neutron, protein or blood related content (e.g. glucose, cholesterol, bilirubin, uric acid or alcohol).

It is noted that the above-mentioned embodiments are merely illustrative, and it's not a limitation of the present invention. As long as the photo detector(s) can receive the light reflected from the needed layer to generate the physiological signals, the quantity of the light emitters, the quantity of the photo detector and the wavelength of the light emitter can be changed according to the designer's consideration.

In one embodiment, the biosensor 110 may combine at least part of the embodiments shown in FIGs. 3-5 to obtain the physiological features of two layers or three layers of the human body. For example, the biosensor 110 may combine the embodiments shown in FIG. 3 and FIG. 4, so that the photo detector (s) 114 can receive the light reflected from the third layer and the second layer to generate the physiological signals corresponding to the third layer and the second layer; the biosensor 110 may combine the embodiments shown in FIG. 3 and FIG. 5, so that the photo detector (s) 114 can receive the light reflected from the third layer and the first layer to generate the physiological signals corresponding to the third layer and the second layer; the biosensor 110 may combine the embodiments shown in FIG. 4 and FIG. 5, so that the photo detector (s) 114 can receive the light reflected from the second layer and the first layer to generate the physiological signals corresponding to the second layer and the first layer; and the biosensor 110 may combine the embodiments shown in FIG. 3, FIG. 4 and FIG. 5, so that the photo detector (s) 114 can receive the light reflected from the third layer, the second layer and the first layer to generate the physiological signals corresponding to the third layer, the second layer and the first layer. These alternative designs shall fall within the scope of the present invention.

Briefly summarized, in the opto-mechanical design of the biosensor of the present invention, by using the LEDs with special wavelengths (e.g. one of the LED emits light having the wavelength greater than 1000 nm) and distance between each of the photo detector and each light emitter, the biosensor can effectively and accurately measure one or more specific layers of a human body.

Those skilled in the art will readily observe that numerous modifications and alterations of the device and method may be made while retaining the teachings of the invention. Accordingly, the above disclosure should be construed as limited only by the metes and bounds of the appended claims.

The present invention may also be defined by means of the following numbered clauses:
1. A biosensor, comprising:
   at least two light emitters, wherein the at least two light emitters comprise a first light emitter and a second light emitter, and the light emitter emits light whose wavelength is greater than 1000 nanometer (nm);
   at least one photo detector, for receiving the light reflected by a human body to generate a plurality of physiological signals, wherein the physiological signals are arranged to obtain at least one physiological feature of the human body.
2. The biosensor of clause 1, wherein both the first light emitter and the second light emitter generate the lights penetrating to a muscle or bone layer of the human body, and the at least one photo detector receives the light reflected by the muscle or bone layer of the human body to generate at least the portion of the physiological signals.
3. The biosensor of clause 1, wherein both the first light emitter and the second light emitter generate the lights penetrating to a subcutaneous tissue layer of the human body, and the at least one photo detector receives the light reflected by the subcutaneous tissue layer of the human body to generate at least the portion of the physiological signals.
4. The biosensor of clause 1, wherein both the first light emitter and the second light emitter generate the lights penetrating to an epidermis/dermis layer of the human body, and the at least one photo detector receives the light reflected by the epidermis/dermis layer of the human body to generate at least the portion of the physiological signals.
5. The biosensor of clause 1, wherein the at least one photo detector receives the light reflected by at least two different layers of the human body to generate at least a portion of the physiological signals, and the at least two different layers comprise two of epidermis/dermis layer, subcutaneous tissue layer and muscle and bone layer.
6. The biosensor of clause 5, wherein the at least one photo detector receives the light reflected by first layer, a second layer and a third layer of the human body to generate the physiological signals, the first layer is the epidermis/dermis layer, the second layer is the subcutaneous tissue layer, and the third layer is the muscle and bone layer.
7. The biosensor of clause 1, wherein a distance between the first light emitter and the at least one photo detector is different from a distance between the second light emitter and the at least one photo detector.
8. The biosensor of clause 1, wherein the at least one photo detector receives the light reflected by an epidermis/dermis layer of the human body to generate at least a portion of the physiological signals, the light reflected by an epidermis/dermis layer is generated from the second light emitter, and a distance between the second light emitter and the at least one photo detector is ranging from 0.1 -10 millimeter (mm) while the second light emitter emits light whose wavelength is ranging from 350 - 600 nm, or the distance between the second light emitter and the at least one photo detector is ranging from 0.1 -5 mm while the second light emitter emits light whose wavelength is ranging from 600 - 1100 nm.
9. The biosensor of clause 1, wherein the at least one photo detector receives the light reflected by an epidermis/dermis layer of the human body to generate at least a portion of the physiological signals, the light reflected by the epidermis/dermis layer is generated from the first light emitter, and a distance between the first light emitter and the at least one photo detector is ranging from 0.1 -10 mm while the first light emitter emits light whose wavelength is ranging from 1100 - 2000 nm.
10. The biosensor of clause 1, wherein the at least one photo detector receives the light reflected by a subcutaneous tissue layer of the human body to generate at least a portion of the physiological signals, the light reflected by the subcutaneous tissue layer is generated from the second light emitter, and a distance between the second light emitter and the at least one photo detector is ranging from 0.1 -10 mm while the second light emitter emits light whose wavelength is ranging from 350 - 600 nm, or the distance between the second light emitter and the at least one photo detector is ranging from 5 -15 mm while the second light emitter emits light whose wavelength is ranging from 600 - 1100 nm.
11. The biosensor of clause 1, wherein the at least one photo detector receives the light reflected by the subcutaneous tissue layer of the human body to generate at least a portion of the physiological signals, the light reflected by the subcutaneous tissue layer is generated from the first light emitter, and a distance between the first light emitter and the at least one photo detector is ranging from 5 -20 mm while the first light emitter emits light whose wavelength is ranging from 1100 - 2000 nm.
12. The biosensor of clause 1, wherein the at least one photo detector receives the light reflected by a muscle/bone layer of the human body to generate at least a portion of the physiological signals, the light reflected by the muscle/bone layer is generated from the second light emitter, and a distance between the second light emitter and the at least one photo detector is ranging from 0.1 -10 mm while the second light emitter emits light whose wavelength is ranging from 350 - 600 nm, or the distance between the second light emitter and the at least one photo detector is ranging from 10 -35 mm while the second light emitter emits light whose wavelength is ranging from 600 - 1100 nm.
13. The biosensor of clause 1, wherein the at least one photo detector receives the light reflected by the muscle/bone layer of the human body to generate at least a portion of the physiological signals, the light reflected by the muscle/bone layer is generated from the first light emitter, and a distance between the first light emitter and the at least one photo detector is ranging from 15 -50 mm while the first light emitter emits light whose wavelength is ranging from 1100 - 2000 nm.
14. An electronic device, comprising:
   a biosensor, comprising:
      at least two light emitters, wherein the at least two light emitters comprise a first light emitter and a second light emitter, and the first light emitter emits light whose wavelength is greater than 1000 nanometer (nm);
      at least one photo detector, for receiving the light reflected by a human body to generate a plurality of physiological signals, wherein the physiological signals are arranged to obtain at least two physiological features of the human body; and
   a processing circuit, coupled to the biosensor, for analyzing the plurality of physiological signals to obtain at least one physiological feature of the human body.
15. The electronic device of clause 14, wherein both the first light emitter and the second light emitter generate the lights penetrating to a muscle or bone layer of the human body, and the at least one photo detector receives the light reflected by the muscle or bone layer of the human body to generate at least the portion of the physiological signals.
16. The electronic device of clause 14, wherein both the first light emitter and the second light emitter generate the lights penetrating to a subcutaneous tissue layer of the human body, and the at least one photo detector receives the light reflected by the subcutaneous tissue layer of the human body to generate at least the portion of the physiological signals.
17. The electronic device of clause 14, wherein both the first light emitter and the second light emitter generate the lights penetrating to an epidermis/dermis layer of the human body, and the at least one photo detector receives the light reflected by the epidermis/dermis layer of the human body to generate at least the portion of the physiological signals.
18. The electronic device of clause 14, wherein at least one of the first light emitter and the second light emitter generates the lights penetrating to a muscle or bone layer of the human body, and the at least one photo detector receives the light reflected by the muscle or bone layer of the human body to generate at least the portion of the physiological signals; and the processing circuit analyzes the plurality of physiological signals to obtain muscle content, muscle density, bone content, bone density or lactate of the human body.
19. The electronic device of clause 14, wherein at least one of the first light emitter and the second light emitter generates the lights penetrating to a subcutaneous tissue layer of the human body, and the at least one photo detector receives the light reflected by the subcutaneous tissue layer of the human body to generate at least the portion of the physiological signals; and the processing circuit analyzes the plurality of physiological signals to obtain body water, fat content, fat density, neutron, protein, glucose, cholesterol, bilirubin, uric acid or alcohol of the human body.
20. The electronic device of clause 14, wherein at least one of the first light emitter and the second light emitter generates the lights penetrating to an epidermis/dermis layer of the human body, and the at least one photo detector receives the light reflected by the epidermis/dermis layer of the human body to generate at least the portion of the physiological signals; and the processing circuit analyzes the plurality of physiological signals to obtain water, collagen, melanin, elastic fiber, neutron, protein, glucose, cholesterol, bilirubin, uric acid or alcohol of the human body.

## Claims

1. A biosensor (110), comprising:
at least two light emitters (112_1 - 112_N), wherein the at least two light emitters (112_1 - 112_N) comprise a first light emitter and a second light emitter, and the light emitter emits light whose wavelength is greater than 1000 nanometer, nm;
at least one photo detector (114), for receiving the light reflected by a human body to generate a plurality of physiological signals, wherein the physiological signals are arranged to obtain at least one physiological feature of the human body.

2. The biosensor (110) of claim 1, wherein both the first light emitter and the second light emitter generate the lights penetrating to a muscle or bone layer of the human body, and the at least one photo detector (114) receives the light reflected by the muscle or bone layer of the human body to generate at least the portion of the physiological signals; and/or
wherein both the first light emitter and the second light emitter generate the lights penetrating to a subcutaneous tissue layer of the human body, and the at least one photo detector (114) receives the light reflected by the subcutaneous tissue layer of the human body to generate at least the portion of the physiological signals; and/or
wherein both the first light emitter and the second light emitter generate the lights penetrating to an epidermis/dermis layer of the human body, and the at least one photo detector (114) receives the light reflected by the epidermis/dermis layer of the human body to generate at least the portion of the physiological signals.

3. The biosensor (110) of claim 1, wherein the at least one photo detector (114) receives the light reflected by at least two different layers of the human body to generate at least a portion of the physiological signals, and the at least two different layers comprise two of epidermis/dermis layer, subcutaneous tissue layer and muscle and bone layer.

4. The biosensor (110) of claim 3, wherein the at least one photo detector (114) receives the light reflected by first layer, a second layer and a third layer of the human body to generate the physiological signals, the first layer is the epidermis/dermis layer, the second layer is the subcutaneous tissue layer, and the third layer is the muscle and bone layer.

5. The biosensor (110) of claim 1, wherein a distance between the first light emitter and the at least one photo detector (114) is different from a distance between the second light emitter and the at least one photo detector (114).

6. The biosensor (110) of claim 1, wherein the at least one photo detector (114) receives the light reflected by an epidermis/dermis layer of the human body to generate at least a portion of the physiological signals, the light reflected by an epidermis/dermis layer is generated from the second light emitter, and a distance between the second light emitter and the at least one photo detector (114) is ranging from 0.1 -10 millimeter, mm, while the second light emitter emits light whose wavelength is ranging from 350 - 600 nm, or the distance between the second light emitter and the at least one photo detector (114) is ranging from 0.1 -5 mm while the second light emitter emits light whose wavelength is ranging from 600 - 1100 nm.

7. The biosensor (110) of claim 1, wherein the at least one photo detector (114) receives the light reflected by an epidermis/dermis layer of the human body to generate at least a portion of the physiological signals, the light reflected by the epidermis/dermis layer is generated from the first light emitter, and a distance between the first light emitter and the at least one photo detector (114) is ranging from 0.1 -10 mm while the first light emitter emits light whose wavelength is ranging from 1100 - 2000 nm.

8. The biosensor (110) of claim 1, wherein the at least one photo detector (114) receives the light reflected by a subcutaneous tissue layer of the human body to generate at least a portion of the physiological signals, the light reflected by the subcutaneous tissue layer is generated from the second light emitter, and a distance between the second light emitter and the at least one photo detector (114) is ranging from 0.1 -10 mm while the second light emitter emits light whose wavelength is ranging from 350 - 600 nm, or the distance between the second light emitter and the at least one photo detector (114) is ranging from 5 -15 mm while the second light emitter emits light whose wavelength is ranging from 600 - 1100 nm.

9. The biosensor (110) of claim 1, wherein the at least one photo detector (114) receives the light reflected by the subcutaneous tissue layer of the human body to generate at least a portion of the physiological signals, the light reflected by the subcutaneous tissue layer is generated from the first light emitter, and a distance between the first light emitter and the at least one photo detector (114) is ranging from 5 -20 mm while the first light emitter emits light whose wavelength is ranging from 1100 - 2000 nm.

10. The biosensor (110) of claim 1, wherein the at least one photo detector (114) receives the light reflected by a muscle/bone layer of the human body to generate at least a portion of the physiological signals, the light reflected by the muscle/bone layer is generated from the second light emitter, and a distance between the second light emitter and the at least one photo detector (114) is ranging from 0.1 -10 mm while the second light emitter emits light whose wavelength is ranging from 350 - 600 nm, or the distance between the second light emitter and the at least one photo detector (114) is ranging from 10 -35 mm while the second light emitter emits light whose wavelength is ranging from 600 - 1100 nm.

11. The biosensor (110) of claim 1, wherein the at least one photo detector (114) receives the light reflected by the muscle/bone layer of the human body to generate at least a portion of the physiological signals, the light reflected by the muscle/bone layer is generated from the first light emitter, and a distance between the first light emitter and the at least one photo detector (114) is ranging from 15 -50 mm while the first light emitter emits light whose wavelength is ranging from 1100 - 2000 nm.

12. An electronic device (100), comprising:
a biosensor (110), comprising:
at least two light emitters, wherein the at least two light emitters comprise a first light emitter and a second light emitter, and the first light emitter emits light whose wavelength is greater than 1000 nanometer, nm;
at least one photo detector (114), for receiving the light reflected by a human body to generate a plurality of physiological signals, wherein the physiological signals are arranged to obtain at least two physiological features of the human body; and
a processing circuit (120), coupled to the biosensor (110), for analyzing the plurality of physiological signals to obtain at least one physiological feature of the human body.

13. The electronic device (100) of claim 12, wherein both the first light emitter and the second light emitter generate the lights penetrating to a muscle or bone layer of the human body, and the at least one photo detector (114) receives the light reflected by the muscle or bone layer of the human body to generate at least the portion of the physiological signals; and/or
wherein both the first light emitter and the second light emitter generate the lights penetrating to a subcutaneous tissue layer of the human body, and the at least one photo detector (114) receives the light reflected by the subcutaneous tissue layer of the human body to generate at least the portion of the physiological signals; and/or
wherein both the first light emitter and the second light emitter generate the lights penetrating to an epidermis/dermis layer of the human body, and the at least one photo detector (114) receives the light reflected by the epidermis/dermis layer of the human body to generate at least the portion of the physiological signals.

14. The electronic device (100) of claim 12, wherein at least one of the first light emitter and the second light emitter generates the lights penetrating to a muscle or bone layer of the human body, and the at least one photo detector (114) receives the light reflected by the muscle or bone layer of the human body to generate at least the portion of the physiological signals; and the processing circuit (120) analyzes the plurality of physiological signals to obtain muscle content, muscle density, bone content, bone density or lactate of the human body; and/or
wherein at least one of the first light emitter and the second light emitter generates the lights penetrating to a subcutaneous tissue layer of the human body, and the at least one photo detector (114) receives the light reflected by the subcutaneous tissue layer of the human body to generate at least the portion of the physiological signals; and the processing circuit (120) analyzes the plurality of physiological signals to obtain body water, fat content, fat density, neutron, protein, glucose, cholesterol, bilirubin, uric acid or alcohol of the human body.

15. The electronic device (100) of claim 12, wherein at least one of the first light emitter and the second light emitter generates the lights penetrating to an epidermis/dermis layer of the human body, and the at least one photo detector (114) receives the light reflected by the epidermis/dermis layer of the human body to generate at least the portion of the physiological signals; and the processing circuit (120) analyzes the plurality of physiological signals to obtain water, collagen, melanin, elastic fiber, neutron, protein, glucose, cholesterol, bilirubin, uric acid or alcohol of the human body.
